# EUROPÄISCHE PATENTANMELDUNG

(11) **EP 1 382 312 A1**
(43) Veröffentlichungstag der Anmeldung: **21.01.2004**
(21) Anmeldenummer: 03015302.7
(22) Anmeldetag: 07.07.2003
(51) Int. Cl.: A61D 3/00

(54) **Verfahren und Vorrichtung zur Immobilisierung und Untersuchung von Lebewesen sowie Verwendung derselben zur optischen Bildgebung**

(30) Priorität: 18.07.2002 DE 10232679
(71) Anmelder: SIEMENS AKTIENGESELLSCHAFT, 80333 München (DE)
(72) Erfinder: Hengerer, Arne, Dr., 91054 Erlangen (DE); Pfister, Marcus, Dr., 91058 Erlangen (DE); Sieber, Heike, 96047 Bamberg (DE)

(57) **Zusammenfassung**

Die vorliegende Erfindung betrifft ein Verfahren und eine Vorrichtung zur Immobilisierung von Lebewesen, um ein zu untersuchendes Lebewesen (8) mittels eines Untersuchungsgeräts (7) während einer vorbestimmbaren Zeitspanne Δt in einem Untersuchungsraum (15) zu untersuchen. Zu diesem Zweck wird das Lebewesen (8) mittels mindestens zweier Schleusen (6a, 6b) in dem Untersuchungsraum (15) während mindestens der für die Untersuchung notwendigen vorbestimmbaren Zeitspanne Δt eingeschlossen. Eine Narkose kann entfallen.

## Beschreibung

Verfahren und Vorrichtung zur Immobilisierung und Untersuchung von Lebewesen sowie Verwendung derselben zur optischen Bildgebung.

Die Erfindung bezieht sich auf ein Verfahren bzw. eine Vorrichtung zur Immobilisierung und Untersuchung von Lebewesen der in den Oberbegriffen der Patentansprüche 1 und 5 genannten Gattung.

Es ist allgemein bekannt, Lebewesen, vor allem Tiere, zu immobilisieren, bevor diese einer Untersuchung zugeführt werden, um zu verhindern, dass die Untersuchungsergebnisse bei beispielsweise Ultraschallaufnahmen, Röntgenaufnahmen oder CT-Aufnahmen durch die Bewegung des Lebewesens verfälscht werden. Auch bei der Untersuchung von Stoffwechselvorgängen auf molekularer Ebene, beispielsweise im lebenden Kleintier, sind optische Bildgebungsgeräte zusammen mit Kontrastmitteln im Einsatz, die im nahen Infrarotbereich fluoreszieren, so dass auch hier das zu untersuchende Lebewesen immobilisiert werden muss. Diese Verfahren finden ihre Anwendungen in zahlreichen Bereichen der Biologie, der Medizin sowie in der Pharmazie, beispielsweise bei der Aufklärung von Signaltransduktionswegen, in der Gentherapie, in der Tumorforschung und bei der Entwicklung von neuen Medikamenten.

Bisher wurden Lebewesen bei entsprechenden Untersuchungsverfahren für die Dauer der Untersuchung anästhesiert und fixiert. Im Gegensatz zu größeren Tieren oder zu Menschen besteht insbesondere bei Kleintieren das Problem, dass hier nur eine geringe Anzahl von Narkoseprotokolle zur Verfügung steht, so dass sich die richtige Dosierung der Anästhetika schwierig gestaltet. Die Dosis des Anästhetikums ist abhängig vom Alter, Geschlecht und der Stoffwechselrate des Tieres, so dass jede Narkose ein erhöhtes gesundheitliches Risiko für das zu untersuchende Lebewesen, insbesondere für ein zu untersuchendes Kleintier, darstellt. Die Narkotisierung erfordert ausserdem eine intensive Überwachung der Vitalparameter, um Komplikationen während der Untersuchung zu vermeiden. Mäuse sind im anästhesierten Zustand beispielsweise nicht mehr in der Lage, ihre Körpertemperatur selbst zu kontrollieren, sie neigen hier zu Unterkühlung. Die Körpertemperatur dieser Tiere wird während der Narkose extern gemessen und beispielsweise mittels eines Wärmebetts oder einer Wärmelampe reguliert.

Die intensive Betreuung der zu untersuchenden Lebewesen während der Untersuchung ist einerseits zeitaufwending, da sie manuell ausgeführt werden muss und andererseits kostenintensiv, da zusätzliche Geräte und Medikamente erforderlich sind. Auch besteht für die zum Teil sehr teuren Versuchstiere ein erhöhtes Gesundheitsrisiko.

Herkömmliche Verfahren mittels Narkose können keinen hohen Durchsatz an Versuchstieren gewährleisten, da eine Automation nur schwer möglich ist. Darüber hinaus stellt das narkotisierte Tier nicht zwingend den tatsächlichen physiologischen Zustand dar, wodurch die Übertragbarkeit der Ergebnisse eines eventuell artifiziellen Systems auf andere Organismen, sowie die Aussagekraft der Ergebnisse reduziert wird.

Aus der DE 21 14 958 ist ein Viehbehandlungsverschlag bekannt, der aus einem Rahmen mit einem Einlass- und einem Auslassgitter für das Vieh besteht, wobei das Vieh mittels eines Bügels innerhalb des Verschlags festgehalten werden kann. Nach Zuführen des Viehs in den Verschlag wird bei geschlossenem Auslass am Einlass eine Schlußstange angebracht, um das Vieh am Zurückdrängen aus dem Verschlag zu hindern.

Aus der DE 31 46 446 ist ein Verfahren und eine Vorrichtung zur Blutentnahme bei einem lebenden Versuchstier bekannt, bei dem das Versuchstier zwischen zwei Platten mittels torartiger Glieder mechanisch festgehalten wird. Desweiteren sind röhren- und/oder tunnelartige Festhalteeinrichtungen genannt, in welche ein Versuchstier eingeschlossen werden kann, um beispielsweise aus dem aus der Einrichtung hervorragenden Schwanz Blut zu entnehmen.

Der Erfindung liegt daher die Aufgabe zugrunde, die Nachteile der vorerwähnten gattungsgemäßen Verfahren und Vorrichtungen zu vermeinden und ein kostengünstiges und nur gering zeitaufwändiges Immobilisierungsverfahren bzw. Vorrichtung anzugeben, die einen hohen Durchsatz an zu untersuchenden Lebewesen ermöglichen. Auch sollen die Untersuchungsergebnisse bei der Anwendung des Verfahrens bzw. der Vorrichtung nicht verfälscht und falls möglich sogar genauer sein als bei den herkömmlichen Immobilisierungssystemen.

Die Erfindung löst die ihr zugrunde liegende Aufgabe durch die kennzeichnenden Merkmale der unabhängigen Patentansprüche 1 und 5. Vorteilhafte Ausgestaltungen der Erfindung sind in den Unteransprüchen gekennzeichnet und dort beschrieben.

Das erfindungsgemäße Verfahren zur Immobilisierung von Lebewesen, um ein zu untersuchendes Lebewesen mittels eines Untersuchungsgeräts während einer vorbestimmbaren Zeitspanne in einem Untersuchungsraum zu untersuchen, nutzt einen Untersuchungsraum, in dem das Lebewesen während mindestens der vorbestimmbaren Zeitspanne eingeschlossen wird. Hierzu dienen insbesondere zwei Schleusen, die den Untersuchungsraum absperren. Der Untersuchungsraum und die mindestens zwei Schleusen sind Teile eines Untersuchungssystems, das weiterhin einen Eingang und einen Ausgang sowie darüber hinaus eventuell noch einen Zuführraum und einen Abführraum aufweist.

Das erfindungsgemäße Verfahren kennzeichnet sich insbesondere durch die folgenden Schritte:
1. Die zweite, dem Ausgang zugewandte Schleuse wird geschlossen.
2. Das Lebewesen wird durch den Eingang zugeführt.
3. Sobald sich das Lebewesen im Untersuchungsraum befindet, der durch die geschlossene zweite Schleuse bereits teilweise verschlossen ist, wird auch die erste, dem Eingang zugewandte Schleuse des Untersuchungsraums geschlossen.
4. Die Untersuchung wird gestartet, sobald sich das Lebewesen im Untersuchungsraum befindet.
5. Die zweite Schleuse wird nach Schließen der ersten Schleuse und nach Ablauf der vorbestimmbaren Zeitspanne wieder geöffnet, so dass das Lebewesen eventuell über den Abführraum und durch den Ausgang das Untersuchungssystem verlassen kann.

Durch das erfindungsgemäße Verfahren ist es möglich, das Lebewesen ohne Narkose und ohne zusätzliche Medikamente in sehr kurzer Zeit zu untersuchen, in dem dieses im Untersuchungsraum, insbesondere zwischen den beiden Schleusen, für eine vorbestimmte Zeitspanne eingesperrt wird. Durch den Wegfall der Anästhesie für das Lebewesen, insbesondere durch den Wegfall einer Narkose für Kleintiere, werden mögliche Komplikationen für das Lebewesen vermindert. Dies führt zu einer Steigerung des Durchsatzes der zu untersuchenden Lebewesen im Untersuchungssystem und somit zu einer Kostenersparnis.

Darüber hinaus wird bei der erfindungsgemäßen Art der Untersuchung von Kleintieren der Kontakt zwischen Mensch und Versuchstier minimiert, wodurch das Infektionsrisiko des Versuchstiers verringert wird. Dies dient einerseits zum Schutz des Menschen vor infektiösen Tieren und andererseits zum Schutz von krankheitsanfälligen Tieren (beispielsweise Nacktmäusen) vor den Keimen des Menschen.

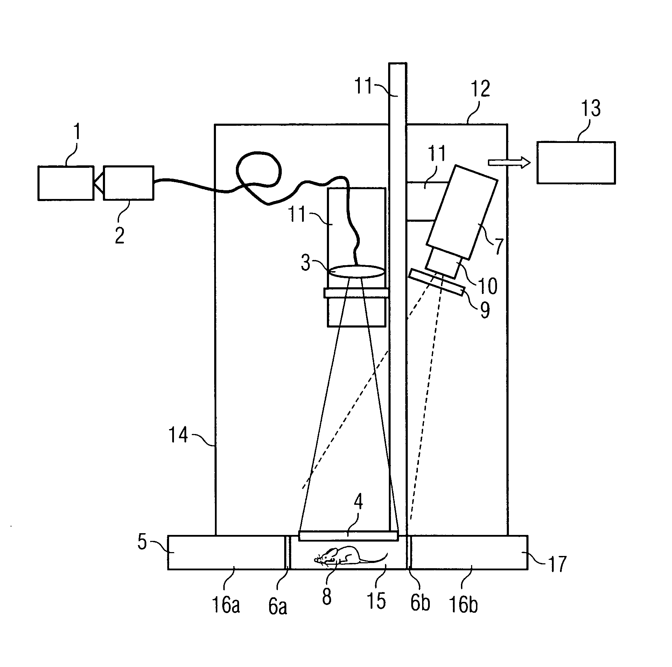

Eine bevorzugte Ausführungsform des Verfahrens und die erfindungsgemäße Vorrichtung wird anhand der Figur 1 näher erläutert:
Figur 1 zeigt schematisch die Anordnung einer Vorrichtung zur Immobilisierung von Lebewesen mit einem Untersuchungsraum 15 und einem Untersuchungsgerät 7, um ein zu untersuchendes Lebewesen 8 während einer vorbestimmbaren Zeitspanne Δt in einem Untersuchungssytem zu untersuchen. Mit Vorteil ist das Untersuchungssystem und das Untersuchungsgerät 7 benachbart angeordnet. Gemäß der erfindungsgemäßen Vorrichtung wird das Lebewesen 8 in dem Untersuchungsraum 15 während mindestens der vorbestimmbaren Zeitspanne Δt eingeschlossen.

Die erfindungsgemäße Vorrichtung weist mindestens zwei Schleusen 6a, 6b auf, die das Lebewesen 8 in dem Untersuchungsraum 15 einschließen sowie eine Zu- und Abführung des Lebewesens 8 ermöglichen. Das Untersuchungssystem weist insbesondere einen Eingang 5, vorzugsweise einen Zuführraum 16a, den Untersuchungsraum 15, vorzugsweise einen Abführraum 16b und einen Ausgang 17 auf, die mit Vorteil einen länglichen Tunnel bilden, das einen Durchmesser aufweist, der die Bewegungsfreiheit des Lebewesens 8 in sämtlichen Richtungen ausser längs des Tunnels weitgehend einschränkt, um eine möglichst verzerrungsfreie Aufnahme des Lebewesens 8 zu ermöglichen.

Zur Untersuchung des Lebewesens mittels eines optischen Bildgebungsgeräts ist das Untersuchungssystem, jedenfalls der Untersuchungsraum 15 und die mindestens zwei Schleusen 6a und 6b, vorzugsweise lichtundurchlässig ausgestaltet, wobei das optische Untersuchungsgerät 7 mittels eines Fensters 4 mit dem Untersuchungsraum 15 in Verbindung steht. Das Fenster 4 ist jedenfalls für die für die Untersuchung notwendigen Strahlen (Lichtstrahlen, Röntgenstrahlen, elektromagnetische Wellen etc.) durchlässig. Für die Anwendung der erfindungsgemäßen Immobilisierungsvorrichtung bei einem bildgebenden Verfahren eignet sich als Untersuchungsgerät 7 beispielsweise eine CCD-Kamera, wobei die Schleusen 6a und 6b als lichtdichte Türen ausgeführt sind. Sobald das Lebewesen 8 im Untersuchungsraum 15 anwesend ist, erfolgt die Untersuchung für eine vorbestimmbare Zeitspanne Δt von vorzugsweise 0,5 s bis 5 s, insbesondere etwa 1 s.

Eine vorteilhafte Verwendung des Verfahrens bzw. der Vorrichtung dient zur Immobilisierung von Tieren bei der optischen in-vivo-Kleintierbildgebung. Dabei wird mittels einer Lichtquelle 1 und eines Lichtfilters 2 Licht im Infrarotbereich durch ein Fenster 4 auf das sich im Untersuchungsraum 15 befindliche und mit einem Kontrastmittel versehene Kleintier geworfen. Die reflektierten Lichtstrahlen, die beispielsweise fluoreszierenden Bereiche des Kleintiers 8 kennzeichnen, werden mittels einer optischen CCD-Kamera detektiert, die die Lichtstrahlen durch einen Bildfilter 9 und eine Bildlinse 10 auffängt. Die digitalen Daten der CCD-Kamera werden an ein entsprechendes Bildverarbeitungssystem 13 weitergeleitet. Linse 3 und Untersuchungsgerät 7 sind mittels Halterungen 11 in einem lichtdichten Gehäuse 14 untergebracht und durch einen Deckel 12 zugänglich.

Das Kleintier 8 wird aus einem Aufbewahrungsraum (beispielsweise Stall, hier nicht gezeigt) durch den Eingang 5 in einen Zuführraum 16a geleitet. Sobald ein zuvor untersuchtes Kleintier 8 sich im Abführraum 16b befindet, wird die zweite Schleuse 6b geschlossen und die erste Schleuse 6a geöffnet. Das sich im Zuführraum 16a befindliche Kleintier 8 kann nun in den Untersuchungsraum 15 gelangen. Sobald sich das Kleintier 8 im Untersuchungsraum 15 befindet, wird die erste Schleuse 6a geschlossen und die Untersuchung aktiviert. Während das Tier im lichtundurchlässigen Tunnel durch das Messfeld des optischen Geräts läuft bzw. sich im Untersuchungsraum 15 befindet, wird automatisch die Analyse durchgeführt. Eine hochsensitive CCD-Kamera ermöglicht Aufnahmezeiten von weniger als 1 s, so dass entsprechende Bewegungen des Kleintiers 8 die Aufnahme bzw. die Untersuchung kaum oder gar nicht stören. Zwischen lichtdichtem Gehäuse 14 und Untersuchungsraum 15 befindet sich beispielsweise ein Fenster 4 aus einem NIR-durchlässigen Material.

Sobald die Untersuchung durchgeführt wurde, d.h. nach der vorbestimmbaren Zeitspanne Δt, öffnet sich die zweite Schleuse 6b und das Kleintier 8 gelangt in den Abführraum 16b und über den Ausgang 17 zurück in den Aufbewahrungsraum (nicht dargestellt).

## Patentansprüche

1. Verfahren zur Immobilisierung und Untersuchung von Lebewesen, wobei das zu untersuchende Lebewesen (8) mittels mindestens zweier Schleusen (6a, 6b) in einem Untersuchungsraum (15) während mindestens einer vorbestimmbaren Zeitspanne (Δt) eingeschlossen wird,
**dadurch gekennzeichnet,**
**dass** der Untersuchungsraum (15) mit einem Untersuchungsgerät (7) verbunden wird, und
**dass** die Untersuchung des Lebewesens (8) mit dem Untersuchungsgerät (7) während der vorbestimmbaren Zeitspanne (Δt) automatisch erfolgt.

2. Verfahren nach Anspruch 1,
**dadurch gekennzeichnet,**
**dass** der Untersuchungsraum (15) und die mindestens zwei Schleusen (6a, 6b) Teile eines Untersuchungssystems sind, das weiterhin einen Eingang (5), einen Abführraum (16b) und einen Ausgang (17) aufweist.

3. Verfahren nach Anspruch 1 oder 2,
**gekennzeichnet durch**,
folgende Schritte:
a) Schliessen der zweiten, dem Ausgang (17) zugewandten Schleuse (6b),
b) Zuführen des Lebewesens (8),
c) Schliessen der ersten, dem Eingang (5) zugewandten Schleuse (6a) sobald sich das Lebewesen (8) im Untersuchungsraum (15) befindet,
d) Öffnen der zweiten Schleuse (6b) nach Schliessen der ersten Schleuse (6a) und nach Ablauf der vorbestimmbaren Zeitspanne (Δt).

4. Verfahren nach einem der vorhergehenden Ansprüche,
**dadurch gekennzeichnet,**
**dass** die Untersuchung gestartet wird, sobald sich das Lebewesen (8) im Untersuchungsraum (15) befindet.

5. Vorrichtung zur Immobilisierung von Lebewesen, mit einem Untersuchungsraum (15) und einem Untersuchungsgerät (7), wobei das zu untersuchende Lebewesen (8) während einer vorbestimmbaren Zeitspanne (Δt) in dem Untersuchungsraum (15) während mindestens einer vorbestimmbaren Zeitspanne (Δt) einschliessbar ist,
**dadurch gekennzeichnet,**
**dass** der Untersuchungsraum (15) mit einem Untersuchungsgerät (7) verbunden ist, und
**dass** die Untersuchung des Lebewesens (8) mit dem Untersuchungsgerät (7) während der vorbestimmbaren Zeitspanne (Δt) automatisch erfolgt.

6. Vorrichtung nach Anspruch 5,
**dadurch gekennzeichnet,**
**dass** mindestens zwei Schleusen (6a, 6b) das Lebewesen (8) in dem Untersuchungsraum (15) einschliessen.

7. Vorrichtung nach einem der Ansprüche 5 oder 6,
**dadurch gekennzeichnet,**
**dass** ein Eingang (5), der Untersuchungsraum (15) und ein Ausgang (17) einen länglichen Tunnel bilden, das einen Durchmesser aufweist, der die Bewegungsfreiheit des Lebewesens (8) ausser längs des Tunnels weitgehend einschränkt.

8. Vorrichtung nach einem der Ansprüche 5 - 7,
**dadurch gekennzeichnet,**
**dass** der Untersuchungsraum (15) und die mindestens zwei Schleusen (6a, 6b) lichtundurchlässig sind und ein optisches Untersuchungsgerät (7) mittels eines Fensters (4) mit dem Untersuchungsraum (15) verbunden ist.

9. Vorrichtung nach einem der Ansprüche 5 - 8,
**dadurch gekennzeichnet,**
**dass** als Untersuchungsgerät (7) eine CCD-Kamera, als vorbestimmbare Zeitspanne (Δt) 0,5s bis 5s, insbesondere etwa 1s, und als Schleusen (6a, 6b) lichtdichte Türen gewählt werden.

10. Verfahren nach einem der Ansprüche 1 bis 4,
**dadurch gekennzeichnet,**
**dass** das Verfahren zur Immobilisierung von Tieren bei der optischen in vivo Kleintierbildgebung verwendet wird.

11. Vorrichtung nach einem der vorhergehenden Ansprüche,
**dadurch gekennzeichnet,**
**dass** die Vorrichtung zur Immobilisierung von Tieren bei der optischen in vivo Kleintierbildgebung verwendbar ist.
